# FASCICULE DE BREVET EUROPEEN

(11) **EP 1 016 711 B1**
(45) Date de publication et mention de la délivrance du brevet: **27.10.2004**
(21) Numéro de dépôt: 99403194.6
(22) Date de dépôt: 17.12.1999
(51) Int. Cl.: C12N 7/06, C12N 7/02, C12N 15/86, C12N 5/00, A61K 48/00, A61K 35/76, A61K 35/12

(54) **Procédé d'inactivation de virus enveloppés dans une préparation virale d'adénovirus**
Verfahren zur Inaktivierung vom umhüllten Viren in einem Viruspräparat mit Adenoviren
Process for inactivating enveloped viruses in a viral composition containing adenoviruses

(30) Priorité: 21.12.1998 FR 9816147
(43) Date de publication de la demande: 05.07.2000
(73) Titulaire: TRANSGENE S.A., 67000 Strasbourg (FR)
(72) Inventeur: Gaillac, David, 94320 Thiais (FR); Koehl, Michel, 67000 Strasbourg (FR)
(74) Mandataire: Warcoin, Jacques

(56) Documents cités:
- EP-A- 0 378 208
- EP-A- 0 812 858
- WO-A-94/28152
- WO-A-98/26048
- WO-A-98/39420

## Description

La présente invention concerne un procédé d'inactivation de virus enveloppés susceptibles de contaminer une préparation virale à base de virus non enveloppés. L'invention a également pour objet une préparation virale essentiellement dépourvue de virus enveloppés et une composition pharmaceutique comprenant une telle préparation virale ainsi que leurs utilisations à des fins thérapeutiques ou prophylactiques. L'invention présente un intérêt tout particulier pour des perspectives de thérapie génique, notamment chez l'homme.

La thérapie génique se définit comme le transfert d'information génétique dans une cellule ou un organisme hôte. Le premier protocole appliqué à l'homme a été initié aux Etats Unis en septembre 1990 sur un patient génétiquement immunodéficient en raison d'une mutation affectant le gène codant pour l'Adénine Désaminase (ADA). Il s'agissait de corriger ou remplacer le gène défectueux dont le dysfonctionnement est à l'origine d'une maladie génétique par un gène fonctionnel. Le succès relatif de cette première expérimentation a encouragé le développement de cette technologie qui a été depuis étendue au traitement d'autres maladies aussi bien génétiques qu'acquises (cancers, maladies infectieuses comme le SIDA...) dans le but de délivrer *in situ* des gènes thérapeutiques améliorant la pathologie. La plupart des stratégies utilisent des vecteurs pour véhiculer le gène thérapeutique vers sa cible cellulaire. De nombreux vecteurs tant viraux que synthétiques ont été développés au cours de ces dernières années et ont fait l'objet de nombreuses publications accessibles à l'homme du métier.

L'intérêt des adénovirus à titre de vecteurs de thérapie génique a déjà été évoqué dans l'art antérieur. Ils infectent de nombreux types cellulaires, aussi bien des cellules en division que quiescentes, sont non intégratifs et peu pathogènes. En outre, ils possèdent un tropisme naturel pour les voies respiratoires. Ces propriétés particulières font des adénovirus des vecteurs de choix pour de nombreuses applications thérapeutiques et même vaccinales.

Le cycle infectieux des adénovirus se déroule en 2 étapes. La phase précoce précède l'initiation de la réplication et permet de produire les protéines précoces régulant la réplication et la transcription de l'ADN viral. La réplication du génome est suivie de la phase tardive au cours de laquelle sont synthétisées les protéines structurales qui constituent les particules virales. L'assemblage des nouveaux virions prend place dans le noyau. Dans un premier temps, les protéines virales s'assemblent de manière à former des capsides vides de structure icosaédrique dans lesquelles le génome est encapsidé. Les adénovirus libérés sont susceptibles d'infecter d'autres cellules permissives.

A titre indicatif, leur génome est constitué d'une molécule d'ADN linéaire et bicaténaire d'environ 36 kb qui porte une trentaine de gènes intervenant dans le cycle viral. Les gènes précoces (E1 à E4 ; E pour early en anglais) sont répartis en 4 régions dispersées dans le génome. Les régions E1, E2 et E4 sont essentielles à la replication virale alors que la région E3 impliquée dans la modulation de la réponse immunitaire anti-adénovirus chez l'hôte ne l'est pas. Les gènes tardifs (L1 à L5 ; L pour late signifiant tardif en anglais) codent majoritairement pour les protéines de structure et recouvrent en partie les unités de transcription précoces. Ils sont pour la plupart transcrits à partir du promoteur majeur tardif MLP (pour Major Late Promoter en anglais). En outre, le génome adénoviral porte à ses extrémités des régions agissant *en cis* essentielles à l'encapsidation constituées de séquences terminales inversées (ITR) situées aux extrémités 5' et 3' et d'une région d'encapsidation qui suit l'ITR 5'.

Les vecteurs adénoviraux actuellement utilisés dans les protocoles de thérapie génique sont dépourvus de la majeure partie de la région E1 afin d'éviter leur dissémination dans l'environnement et l'organisme hôte. Des délétions supplémentaires dans la région E3 permettent d'accroître les capacités de clonage. Des vecteurs dîts de seconde génération sont également disponibles. Ils conservent les régions *en cis* (ITRs et séquences d'encapsidation) essentielles à l'encapsidation mais comportent des modifications génétiques supplémentaires visant à réduire l'expression *in vivo* de certains gènes viraux susceptible de nuire à la persistance des cellules transduites et à l'expression stable du transgène (voir par exemple les demandes internationales WO94/28 152 et WO97/04119). A cet égard, un vecteur minimum, déficient pour l'ensemble des fonctions adénovirales, représente une alternative de choix.

Pour des raisons évidentes de sécurité, il est important d'obtenir des préparations virales dépourvues de contaminants potentiellement nocifs. Les adénovirus recombinants sont habituellement produits dans une lignée cellulaire complémentant les fonctions défectives. Après culture, les cellules infectées sont récoltées, lysées et les particules virales sont purifiées à partir du lysat cellulaire. La majorité des équipes travaillant dans ce domaine s'est intéressée à réduire les contaminants moléculaires (protéiques, ADN, minéraux, toxines...) en mettant en oeuvre des techniques d'ultracentrifugation sur gradient de chlorure de césium ou de chromatographie. Mais, la contamination potentielle par d'autres types de virus demeure à ce jour non résolue. A cet égard, les risques pathologiques associés aux virus enveloppés ne sont pas sans conséquences puisqu'ils peuvent conduire à des cancers, hépatites, au SIDA...etc. Il est donc crucial que les préparations de virus recombinants destinées à un usage humain soient dépourvues de virus enveloppés infectieux.

Or, les sources de contamination sont nombreuses tout au long du procédé qui conduit à la préparation des virus d'intérêt. Outre la contamination accidentelle, les lignées cellulaires utilisées pour propager les virus d'intérêt peuvent comprendre, intégré dans leurs chromosomes un certain nombre de génomes rétroviraux (provirus). Ceux-ci peuvent être activés en réponse à certaines conditions de culture pour générer des virus enveloppés infectieux. De plus, les milieux de cultures contiennent fréquemment du sérum d'origine animale qui est une source majeure de virus enveloppés. De plus, les opérateurs, l'environnement et le matériel à usage multiple (fermenteur, homogéneiseur, colonne de chromatographie...) peuvent également contribuer à la contamination. Ces contaminants sont appelés agents étrangers et concernent les virus enveloppés mais également les bactéries et les cellules.

Un procédé d'inactivation des virus enveloppés par un mélange de tri(n-butyl) phosphate (TNBP) a déjà été mis en oeuvre pour la préparation de protéines et dérivés sanguins (concentrés plaquettaires, cryoprécipités, produits de fractionnement....) où la contamination par les virus de l'hépatite B constitue un problème majeur de santé publique Un tel procédé n'a jamais été appliqué à une préparation de virus non enveloppés où la contamination par les virus enveloppés s'oppose à la sécurité des lots cliniques

Contrairement au procédé de l'art antérieur applicable aux compositions protéiques, se pose le problème particulier de la co-existence de deux types viraux dans la même préparation, d'une part les virus non enveloppés que l'on souhaite préserver et les virus enveloppés que l'on cherche à inactiver. Un procédé selon l'invention doit concilier ces deux préalables. D'une manière générale, les virus présentent une architecture structurale complexe et l'intégrité de la particule virale est essentielle pour l'infectivité et la pénétration dans les cellules hôtes. A cet égard, les adénovirus sont composés d'une molécule d'ADN associée à des protéines et entourée d'une capside icosaédrique. La capside est formée de capsomères comprenant 720 hexons et 60 pentons auxquels sont associés des monomères de polypeptides IIIa, VI et IX qui stabilisent la structure. Liée à la sous unité penton et sortant à l'extérieur de la capside, se trouve la fibre trimérique qui permet l'attachement initial du virus à sa cellule cible. Une capside adénovirale légèrement altérée peut avoir un effet néfaste sur l'infectivité virale. On peut illustrer la fragilité des adénovirus en mentionnant le fait qu'une exposition prolongée à une température supérieure à 37°C suffit à réduire le pouvoir infectieux souvent de plusieurs logs.

On a maintenant mis au point un procédé d'inactivation de virus enveloppés dans une préparation contenant des adénovirus recombinants à titre de principes actifs qui utilise le solvant tri-n-butyl phosphate (TNBP). Par ailleurs, l'effet de différentes variables a été étudié afin de définir les conditions expérimentales les plus adaptées pour préserver l'infectivité des adénovirus recombinants et pour s'intégrer dans un procédé de purification global. Les exemples qui suivent montrent que l'action de 0,1 à 0,6 % de TNBP et de 1 % à 2 % de Tween 80 pendant 4h à température ambiante permet de réduire de manière significative la quantité de virus enveloppés (réduction d'au moins un facteur 4 logs) tout en respectant l'intégralité des particules adénovirales (rendement d'au moins 80%, voire supérieur à 100 %). On a également mis en évidence l'effet bénéfique du procédé selon l'invention pour réduire les agrégats qui se forment spontanément entre les virions et nuisent à l'infectivité des virus.

C'est pourquoi la présente invention a pour objet un procédé d'inactivation de virus enveloppés contaminant une préparation virale contenant majoritairement des adénovirus, dans lequel :
- on introduit dans ladite préparation virale une quantité suffisante de solvant tri-n butyl phosphate (TNBP) comprise entre 0,05 % et 1 % (volume/volume),
- on laisse agir ledit TNBP à une température comprise entre + 4°C et + 37°C, à un pH compris entre 6,5 et 8,5, pendant un temps suffisamment long pour réduire de manière significative la quantité de virus enveloppés contaminant ladite préparation virale,
ledit procédé d'inactivation conservant le pouvoir infectieux desdits adénovirus.

Les « virus enveloppés » et « non enveloppés » sont largement définis dans les ouvrages de base de virologie. Brièvement, les virus enveloppés présentent à leur surface une enveloppe composée d'une couche ou bicouche lipidique et de protéines associées. Sa composition est due au fait qu'elle se forme lors du bourgeonnement des virus à travers la membrane cellulaire. Le terme membrane cellulaire inclut la membrane plasmique et celles des autres organelles cellulaires telles que le réticulum endoplasmique, l'appareil de Golgi, le noyau. Par contraste, un virus non enveloppé ne possède pas de lipide à sa surface et est entouré par une capside protéique.

Une « préparation virale » contient majoritairement un ou plusieurs virus non enveloppé(s) dans un milieu aqueux (milieu de culture, milieu tamponné, solution de formulation...). Le terme « virus » inclut les virus sauvages, mutants et recombinants (comportant au moins un gène d'intérêt). Une préparation virale est habituellement produite par introduction de l'ADN du virus non enveloppé porté par un ou plusieurs fragments dans une lignée cellulaire appropriée ou par l'infection de la lignée par un prestock viral. Puis la lignée transfectée infectée est cultivée et on récolte des particules virales produites des cellules productrices et/ou du surnageant de culture.

On indique que dans le cadre de la présente invention, la préparation virale peut également être soumise à une ou plusieurs étapes de purification visant à atteindre des niveaux de pureté compatibles avec la qualité pharmaceutique requise du produit viral (élimination au moins en partie des contaminants de type protéines, toxines, acides nucléiques....). La purification peut être effectuée par des techniques de centrifugation sur gradient de chlorure de césium ou de chromatographie telles que celles détaillées ci-après.

Un mode de réalisation avantageux de la présente invention consiste en la mise en oeuvre d'un adénovirus défectif pour la réplication dans lequel une ou plusieurs fonctions virales essentielles sont rendues non fonctionnelles par mutation (addition, délétion et/ou substitution d'un ou plusieurs nucléotides continus ou non).

Le procédé d'inactivation selon l'invention vise à réduire ou éliminer les virus enveloppés susceptibles de contaminer une préparation virale comprenant un ou plusieurs adénovirus d'intérêt.

Le terme « inactivation » peut se définir comme une réduction significative ou totale de l'infectivité du (ou des) virus enveloppé(s) contaminant(s) la préparation virale d'intérêt. Aux fins de la présente invention, l'infectivité est réduite d'un facteur d'au moins 2 logs, avantageusement d'au moins 3 logs, de préférence, d'au moins 4 logs et d'une manière tout à fait préférée d'au moins 7 logs. L'inactivation des virus enveloppés peut être évaluée selon les techniques de l'art, par exemple par microscopie électronique, HPLC, méthodes de biologie moléculaire (PCR), méthodes de titration du titre viral, fluorescence, méthodes immunologiques (ELISA, RIA...), méthodes immuno-enzymatiques permettant la détection d'un ou plusieurs polypeptides viraux (Western....), mesure de l'activité réverse-transcriptase notamment pour les rétrovirus....

Aux fins de la présente invention, le solvant peut être introduit dans la préparation virale immédiatement après la récolte des virus non enveloppés (préparation virale non purifiée) ou à une étape quelconque de sa purification.

Dans le cadre de la présente invention, le terme « solvant » désigne toute substance, solution ou composition capable de solubiliser un lipide ou dissocier un constituant comprenant un ou plusieurs lipides. Dans le cas présent, un solvant en usage dans le procédé selon l'invention est plus particulièrement destiné à dissocier une enveloppe virale. Bien que tout solvant puisse être envisagé, on préfère néanmoins mettre en oeuvre l'Hecameg (Interchim référence UP785480), l'éther ou encore un alkyl phosphate, seul ou en combinaison. La combinaison peut associer des solvants de même famille chimique (par exemple deux alkyl-phosphates) ou de familles différentes (éther et alkyl phosphate). Dans le cadre de la présente invention, le solvant est plus particulièrement choisi parmi le groupe constitué par les di-alkyl phosphates et les tri-alkyl phosphates. Avantageusement, chacun des groupes alkyls du di-alkyl ou tri-alkyl phosphate comprend de façon indépendante de 1 à 10 atomes de carbone. On indique que le ou les groupes alkyl peuvent être sous forme linéaire ou branchée (isoalkyl) et peuvent éventuellement être substitués. Il s'agit de préférence d'un tri-alkyl phosphate dont chacun des 3 groupes alkyls comprend de façon indépendante de 2 à 8 atomes de carbone et, de manière tout à fait préférée, de 3 à 5. A titre purement illustratif, on peut citer le tri-(n-butyl) phosphate (TNBP), le tri-(t-butyl) phosphate, le tri-(n-hexyl) phosphate, le tri-(2-ethylhexyl) phosphate, le tri-(n-decyl) phosphate. Un solvant particulièrement préféré est le tri-n butyl phosphate.

La quantité de solvant à employer dans le procédé selon l'invention doit être suffisante pour réduire de manière significative l'infectivité des virus enveloppés contaminant la préparation virale d'intérêt. Bien entendu, ladite quantité peut varier en fonction de certains paramètres du procédé selon l'invention (volume de la préparation virale, taux de contamination, type de virus enveloppés, état de purification de la préparation virale...etc). L'homme de l'art est en mesure d'adapter la quantité de solvant nécessaire aux conditions expérimentales précises. Avantageusement, le solvant est utilisé à une concentration finale comprise entre 0,001 % et 10 % (1% correspondant à 1ml d'une solution mère de solvant d'une pureté supérieure à 99 % ou à 1g de solvant pur pour un volume total de 100 ml). Selon un mode de réalisation préférentiel, le solvant introduit dans ladite préparation virale est du tri-(n-butyl) phosphate et en une quantité comprise entre 0,05 % et 1 %, de manière préférée, entre 0,1% et 0,6% et, de manière tout à fait optimale, de 0,3%.

Selon un mode de réalisation optionnel mais néanmoins avantageux, le procédé d'inactivation de virus enveloppés selon l'invention est réalisé en présence d'un détergent, surfactant ou molécule amphiphile, de préférence non ionique. Ces termes regroupés ci-après sous la dénomination d'agent de solubilisation, désignent toute substance, solution ou composition facilitant la solubilité d'une autre substance, solution ou composition dans un milieu où cette dernière n'est pas ou peu soluble ou encore facilitant son accessibilité aux virus enveloppés. Conformément aux buts poursuivis par la présente invention, l'agent de solubilisation est destiné à améliorer la solubilité ou l'accessibilité du solvant vis à vis des virus enveloppés présents dans la préparation virale d'intérêt dans le but d'accroître l'efficacité du procédé selon l'invention. Bien entendu, le solvant et l'agent de solubilisation peuvent être introduits individuellement dans la préparation virale (l'agent de solubilisation préalablement ou après le solvant) ou encore de manière simultanée. Notamment lorsque le procédé d'inactivation selon l'invention est mis en oeuvre sur une préparation virale en cours de purification, il peut être avantageux d'introduire l'agent de solubilisation dès les premières étapes de purification puis d'introduire le solvant lors du procédé selon l'invention. Eventuellement, des étapes subséquentes de purification pourront parfaire la pureté de la préparation virale, notamment en éliminant du produit final le solvant et, le cas échéant, l'agent de solubilisation utilisés.

Bien que le choix de l'agent de solubilisation ne soit pas limité, on peut citer en particulier les dérivés polyoxyéthylènes d'acides gras ou de leurs esters. Les agents de solubilisation préférés incluent le Tween (notamment le Tween 20 ou 80), le Triton (notamment X-100), le PEG (notamment le PEG 400), le sodium cholate, le sodium déoxycholate, l'octyl β-D glucopyranoside et le N dodécyl N, N diméthyl 2-ammonio 1-éthane sulphonate. Le Tween 80 est tout à fait préféré. La combinaison TNBP et Tween 80 est préférentielle dans le cadre de l'invention.

Dans le cas où ce mode de réalisation est retenu, la concentration finale d'agent de solubilisation à employer peut varier dans une large gamme. A titre indicatif, elle peut être comprise entre 0,001% et 10%, en particulier entre 0,01 % et 5 % et, de préférence entre 0,1 % et 2%. S'agissant du Tween 80, la concentration optimale est comprise entre 0,5 % et 2 %. Les combinaisons TNBP 0,6 % et Tween 80 2 % ainsi que TNBP 0,3 % et Tween 80 1 % sont particulièrement préférées.

Par ailleurs, le procédé selon l'invention peut également être réalisé en présence d'une ou plusieurs autres substances améliorant l'efficacité du solvant vis à vis des virus enveloppés, sa stabilité ou sa solubilité ou réduisant des activités interférantes susceptibles de nuire à l'inactivation des virus enveloppés et/ou l'infectivité des virus non enveloppés. A cet égard, on peut citer notamment les anti-protéases. Ce mode de réalisation est particulièrement approprié à la mise en oeuvre d'un procédé utilisant l'Hecameg à titre de solvant.

La température à laquelle est mis en oeuvre le procédé selon l'invention est comprise entre -5 et +50°C. Cependant, afin de garantir l'infectivité des virus non enveloppés de la préparation virale, on préfère une température comprise entre +4°C et +37°C et, plus particulièrement, entre +15°C-et +25°C, la température ambiante étant tout à fait appropriée.

Le procédé selon l'invention est réalisé à un pH compris entre environ 5 et environ 9. Mais, on préférera opérer à un pH compris entre 6,5 et 8,5 et, de préférence, à un pH de 8,5. L'homme du métier est en mesure d'ajuster le pH par utilisation de solutions, tamponnées ou par ajout de bases ou acides pour respectivement augmenter ou réduire le pH selon les besoins.

Dans le cadre du procédé selon l'invention, le temps de réaction entre le solvant en présence éventuellement de l'agent de solubilisation et la préparation virale peut varier en fonction de différents paramètres (volume de la préparation virale, types de virus enveloppés, température de réaction...etc). Le temps de réaction approprié aux conditions expérimentales peut être facilement déterminé par l'homme de l'art à l'aide de simples essais comparatifs. A titre indicatif, le temps de réaction est compris entre 15 min et 24h, avantageusement entre 30 min et 12h et, de manière préférée, entre 1h et 5h. L'allongement du temps de réaction peut être considéré pour des volumes de préparation virale particulièrement importants ou une température de réaction basse. Par ailleurs, dans le cas où une réduction du temps de réaction est recherchée, l'homme du métier est capable de déterminer la hausse appropriée de la température de réaction.

Préférentiellement, le procédé selon l'invention est réalisé sous agitation. En effet, on a observé que le rendement en virus non enveloppés est accru dans ces conditions. Bien que le choix de la vitesse d'agitation soit très large, il est préférable d'opérer à une vitesse d'agitation comprise entre environ 50 et environ 5000 tours/min, avantageusement entre environ 100 et environ 2000 tours/min et, de préférence entre environ 150 et environ 500 tours/min. On peut avoir recours à un agitateur magnétique ou tout autre appareillage approprié (par exemple cuve munie d'agitateurs à hélices ou pales).

Enfin, il est préférable d'effectuer le procédé selon l'invention dans des conditions de conductivité comprises entre 5 et 500 mS/cm, avantageusement entre 10 et 200 mS/cm et, de préférence, entre 10 et 100 mS/cm. Ces conditions sont avantageuses pour préserver l'infectivité des virus non enveloppés d'intérêt.

Par ailleurs, le procédé selon l'invention peut concerner un ou plusieurs types de virus enveloppés issus de sources variées, comme par exemple la matière première, la matière biologique, l'environnement ou les opérateurs intervenant au cours de la préparation et de la purification des virus non enveloppés d'intérêt. De préférence, le procédé de l'invention est particulièrement utile pour inactiver les virus enveloppés pathogènes pour l'homme. Parmi ceux-ci, on peut citer les virus de l'hépatite, les rétrovirus, le virus Epstein Barr, les cytomégalovirus, les virus de l'herpès, les rhabdovirus, les myxovirus, les paramyxovirus, les orthomyxovirus, les arénavirus et les coronavirus. Dans le cadre de la présente invention, le procédé de l'invention s'adresse plus particulièrement aux rétrovirus et aux virus de l'hépatite. La validation du procédé selon l'invention peut être réalisée en introduisant dans une préparation virale d'intérêt une quantité connue de virus enveloppés particulièrement stables contre l'inactivation, comme par exemple le BVD (bovine viral diarrhea), le PRV (pseudorabies virus), le VSV (Vesicular stomatitis virus), les rétrovirus ou le HSV (herpès simplex virus). Le procédé d'inactivation de l'invention est validé lorsque la concentration et/ou l'infectivité des virus enveloppés «tests » est réduite de manière significative, c'est à dire d'au moins 4 logs. En outre, dans la mesure où le procédé d'inactivation de l'invention est intégré dans un procédé global de préparation d'une préparation virale, il est également possible de prévoir une validation globale permettant de quantifier l'inactivation résultant par l'ensemble des étapes du procédé de préparation. Un exemple de validation de l'étape d'inactivation est fourni ci-après.

Le procédé d'inactivation selon l'invention s'applique à une préparation virale comprenant des adénovirus d'intérêt. Le procédé de l'invention est tout particulièrement adapté à la préparation d'adénovirus recombinants défectifs pour la réplication. « Recombinant » fait référence à la présence d'un ou plusieurs gène(s) d'intérêt placé(s) sous le contrôle des éléments appropriés à son (leur) expression dans une cellule hôte. « Défectifs pour la réplication » signifie incapable de réplication autonome dans une cellule hôte (en l'absence de complémentation).

Avantageusement, le gène d'intérêt code pour un ARN anti-sens, un ribozyme, ou encore un polypeptide d'intérêt. Il peut être issu d'un organisme eucaryote, d'un procaryote d'un parasite ou d'un virus autre qu'un adénovirus. Il peut être isolé par toute technique conventionnelle dans le domaine de l'art (par clonage, PCR, synthèse chimique....). Il peut être de type génomique (comportant tout ou partie de l'ensemble des introns), de type ADN complémentaire (ADNc, dépourvu d'intron) ou de type mixte (minigène). Par ailleurs, le polypeptide pour lequel il code peut être (i) intracellulaire, (ii) incorporé dans la membrane de la cellule hôte ou (iii) secrété. Il peut s'agir d'un polypeptide tel que trouvé dans la nature (natif), d'une portion de celui-ci (tronqué), d'un mutant présentant notamment des propriétés biologiques améliorées ou modifiées ou encore d'un polypeptide chimère provenant de la fusion de séquences d'origines diverses.

Parmi les polypeptides d'intérêt utilisables, on peut citer plus particulièrement les chémokines (MIP-1α MIP-1β, RANTES, DC-CK1, MDC, MCP1 (protéine de chémoattraction des monocytes), IP10....), les cytokines (interféron α, β ou γ, interleukine (IL), notamment l'IL-2, l'IL-6, l'IL-10 ou encore l'IL-12, facteur stimulateur de colonies (GM-CSF, C-CSF, M-CSF)...), les récepteurs cellulaires (notamment reconnus par le virus HIV), les ligands de récepteur, les facteurs de coagulation (Facteur VIII, Facteur IX, thrombine, protéine C), les facteurs de croissance, les facteurs proangiogéniques (FGF pour Fibroblast Growth Factor, VEGF pour Vascular Endothelial Growth Factor, SH/HGF pour scatter factor/Hepatocyte growth factor, TGF pour transforming growth factor, TNF pour facteur nécrosant des tumeurs, angiopoïétine), les enzymes (uréase, rénine, métalloprotéinase, nitric oxide synthétase NOS, SOD, catalase, lécithine cholestérol acyl transférase LCAT...), les inhibiteurs d'enzyme (al-antitrypsine, antithrombine III, inhibiteur de protéase virale, PAI-1 pour plasminogen activator inhibitor), les antigènes du complexe majeur d'histocompatibilité de classe I ou II ou polypeptides agissant sur l'expression des gènes correspondants, les antigènes (ou peptides antigéniques) capables de générer une réponse immunitaire, les polypeptides capables d'inhiber une infection virale, bactérienne ou parasitaire ou son développement, les polypeptides à effet antitumoral (produits d'expression des gènes suppresseurs de tumeurs, antigènes associés aux tumeurs, ...) les polypeptides agissant positivement ou négativement sur l'apoptose (Bax, Bcl2, BclX...), les agents cytostatiques (p21, p 16, Rb), les immunoglobulines en totalité ou en partie (Fab, ScFv...), les toxines, les immunotoxines, les apolipoprotéines (ApoAI, ApoAIV, ApoE...), les produits cytotoxiques, les facteurs anti-angiogéniques (angiostatine, endostatine, PF-4...), les marqueurs (β-galactosidase, luciférase, green fluorescent protein) ou tout autre polypeptide ayant un effet thérapeutique pour l'affection ciblée.

Plus précisément, dans le but de traiter un dysfonctionnement héréditaire, on utilisera une copie fonctionnelle du gène défectueux, par exemple un gène codant pour le facteur VIII ou IX dans le cadre de l'hémophilie A ou B, la dystrophine (ou minidystrophine) dans le cadre des myopathies de Duchenne et Becker, l'insuline dans le cadre du diabète, la protéine CFTR (Cystic Fibrosis Transmembrane Conductance Regulator) dans le cadre de la mucoviscidose. S'agissant d'inhiber l'initiation ou la progression de tumeurs ou cancers, on mettra de préférence en oeuvre un gène d'intérêt codant pour un ARN anti-sens, un ribozyme, un produit cytotoxique (thymidine kinase de virus simplex de l'herpès 1 (TK-HSV-1), ricine, toxine cholérique, diphtérique, produit des gènes de levure *FCY1* et *FUR1* codant pour l'uracyle phosphoribosyl transférase et la cytosine désaminase.....), une immunoglobuline, un inhibiteur de la division cellulaire ou des signaux de transduction, un produit d'expression d'un gène suppresseur de tumeur (p53, Rb, p73, DCC....), un polypeptide stimulateur du système immunitaire, un antigène associé à une tumeur (MUC-1, BRCA-1, antigènes précoces ou tardifs d'un virus à papillome), éventuellement en combinaison avec un gène de cytokine. Enfin, dans le cadre d'une thérapie anti-HIV, on peut avoir recours à un gène codant pour un polypeptide immunoprotecteur, un épitope antigénique, un anticorps (2F5; Buchacher et al., 1992, Vaccines *92*, 191-195), le domaine extracellulaire du récepteur CD4 (sCD4 ; Traunecker et al., 1988, Nature *331*, 84-86) une immunoadhésine (par exemple un hybride CD4-immunoglobuline IgG ; Capon et al., 1989, Nature *337*, 525-531 ; Byrn et al., 1990, Nature *344*, 667-670), une immunotoxine (par exemple fusion de l'anticorps 2F5 ou de l'immunoadhésine CD4-2F5 à l'angiogénine ; Kurachi et al., 1985, Biochemistry *24*, 5494-5499), un variant trans-dominant (EP 0614980, WO95/16780), un produit cytotoxique tel que l'un de ceux mentionné ci-dessus ou encore un IFNα ou β.

Un des gènes d'intérêt peut également être un gène de sélection permettant de sélectionner ou identifier les cellules transfectées ou transduites. On peut citer les gènes *néo* (codant pour la néomycine phosphotransférase) conférant une résistance à l'antibiotique G418, *dhfr* (Dihydrofolate Réductase), CAT (Chloramphenicol Acetyl transférase), *pac* (Puromycine Acétyl-Transferase) ou encore *gpt* (Xanthine Guanine Phosphoribosyl Transferase). D'une manière générale, les gènes de sélection sont connus de l'homme de l'art.

Généralement, le ou les génes d'intérêt sont placés sous le contrôle des éléments de régulation permettant leur expression dans la cellule ou l'organisme hôte. Il s'agit de l'ensemble des éléments génétiques permettant la transcription d'un gène d'intérêt en ARN et la traduction d'un ARNm en polypeptide. Parmi ceux-ci, le promoteur revêt une importance particulière. Il peut être isolé d'un gène quelconque d'origine eucaryote ou même virale et peut être constitutif ou régulable. Alternativement, il peut s'agir du promoteur naturel du gène en question. Par ailleurs, il peut être modifié de manière à améliorer l'activité promotrice, supprimer une région inhibitrice de la transcription, rendre un promoteur constitutif régulable ou *vice versa,* introduire un site de restriction.... On peut mentionner, à titre d'exemples, les promoteurs eucaryotes des gènes PGK (Phospho Glycérate Kinase), MT (metallothioneine ; Mc Ivor et al., 1987, Mol. Cell Biol. *7*, 838-848), SRα (Takebe et al., 1988, Mol. Cell. Biol. *8*, 466-472), le promoteur précoce du virus SV40 (Simian Virus), le LTR du RSV (Rous Sarcoma Virus), le promoteur TK-HSV-1, le promoteur précoce du virus CMV (Cytomegalovirus) et les promoteurs adénoviraux E1A et MLP.

Un promoteur en usage dans la présente invention peut également stimuler l'expression dans une cellule tumorale ou cancéreuse. On peut citer notamment les promoteurs des gènes MUC-1 surexprimé dans les cancers du sein et de la prostate (Chen et al., 1995, J. Clin. Invest. *96*, 2775-2782), CEA (pour carcinoma embryonic antigen) surexprimé dans les cancers du colon (Schrewe et al., 1990, Mol. Cell, Biol. *10*, 2738-2748), tyrosinose surexprimé dans les mélanomes (Vile et al., 1993, Cancer Res. *53*, 3860-3864), ERB-2 surexprimé dans les cancers du sein et du pancréas (Harris et al., 1994, Gene Therapy *1*, 170-175) et α-fétoprotéine surexprimée dans les cancers du foie (Kanai et al., 1997, Cancer Res. *57*, 461-465). Un promoteur régulable par des substances hormonales ou exogènes (hormones stéroïdiennes, tétracycline...etc) est également envisageable (Saez et al., 1997, Current Opinion in Biotechnology *8*, 608-616)

On peut aussi avoir recours à un promoteur tissu-spécifique. A titre purement illustratif, on peut citer les promoteurs foie-spécifiques (des gènes α-1 antitrypsine, albumine, FIX, ApoAI...), poumon-spécifiques (des gènes CFTR, surfactant), lymphocyte-spécifiques (immunoglobuline) et muscle-spécifiques (β-actine, Tabin et al., 1982, Mol. Cell Biol. *2*, 426-436 ; SM22 ; Moessler et al., 1996, Devetopment *122*, 2415-2425 et desmine, Li et al., 1989, Gene *78*, 243-254).

Par ailleurs, les éléments de régulation peuvent, en outre, inclure des éléments additionnels améliorant l'expression ou le maintien dans la cellule hôte du gène d'intérêt (origine de réplication, éléments d'intégration dans le génome cellulaire, séquences introniques, séquences poly A de terminaison de la transcription, leaders tripartites...). Ces éléments sont connus de l'homme de l'art. En outre, le gène d'intérêt peut également comporter en amont de la région codante une séquence codant pour un peptide signal permettant sa sécrétion de la cellule hôte. Le peptide signal peut être celui du gène en question ou hétérologue (issu d'un gène quelconque sécrété ou synthétique).

Le gène d'intérêt peut être inséré à un endroit quelconque du génome du virus non enveloppé, avantageusement en remplacement de la région E1 ou E3 lorsqu'il s'agit d'un adénovirus. Lorsque le vecteur adénoviral recombinant comporte plusieurs gènes d'intérêt, ceux-ci peuvent être placés sous le contrôle des même éléments génétiques (cassette polycistronique utilisant un site interne d'initiation de la traduction de type IRES pour réinitier la traduction du second cistron) ou d'éléments indépendants. Dans ce cas, ils peuvent être insérés dans une même région virale (par exemple en remplacement de E1) ou dans des régions différentes (par exemple en remplacement de E1 et d'une autre région délétée).

Un virus défectif peut être obtenu par mutation non fonctionnelle ou par délétion en totalité ou en partie d'une région essentielle à la réplication virale. On aura de préférence recours à un vecteur adénoviral dépourvu de tout ou partie d'au moins une région essentielle à la réplication sélectionnée parmi les régions E1, E2, E4 et L1 à L5, afin d'éviter sa propagation au sein de l'organisme hôte ou de l'environnement. Une délétion de la majorité de la région E1 est préférée. Avantageusement, elle s'étend des nucléotides (nt) 454 à 3328 mais peut également englober des séquences additionnelles en 5' et/ou en 3' à la condition de ne pas interférer avec la fonction d'encapsidation. De préférence, le gène pIX n'est pas indu dans la délétion de E1. Une délétion s'étendant jusqu'au nt 3510 répond à ces critères.

En outre, la délétion de E1 peut être combinée à d'autres modification(s) touchant notamment les régions E2, E4, L1, L2, L3, L4 et/ou L5, dans la mesure où les fonctions essentielles défectives sont complémentées en *trans* au moyen d'une lignée de complémentation et/ou d'un virus auxiliaire. A cet égard, on peut avoir recours aux vecteurs de seconde génération défectifs pour les fonctions E1 et E4 ou E1 et E2 (voir par exemple les demandes internationales WO94/28152 et WO97/04119). Pour illustrer ce mode de réalisation, on peut citer un vecteur combinant une délétion au sein de la région E1 et une mutation thermosensible affectant le gène DBP (pour DNA Binding Protein en anglais) de la région E2A (Ensinger et al., 1972, J. Virol. *10*, 328-339) ou une délétion de cette dernière. Pour ce qui est de la région E4, elle peut être délété en totalité ou en partie. Une délétion partielle de la région E4 à l'exception des séquences codant pour les cadres de lecture ouverts (ORF) 3 et/ou 6/7 est avantageuse dans la mesure où elle ne nécessite pas de complémentation de la fonction E4 (Ketner et al., 1989, Nucleic Acids Res. *17*, 3037-3048). Une autre alternative consiste à maintenir dans le squelette adénoviral les séquences de E4 codant pour les ORFs-3 et 4 ou pour les ORFs 3, 6 et 7 qui ont un effet bénéfique pour l'expression du gène d'intérêt.

Dans le but d'augmenter les capacités de clonage, le vecteur adénoviral recombinant peut en outre être dépourvu de tout ou partie de la région E3 non essentielle. Selon cette alternative, il peut être intéressant de conserver néanmoins les séquences E3 codant pour les polypeptides permettant l'échappement au système immunitaire de l'hôte, notamment la glycoprotéine gpl9k (Gooding et al., 1990, Critical Review of Immunology *10*, 53-71). Selon une autre alternative, on peut employer un vecteur adénoviral minimal retenant essentiellement les ITRs (Inverted Terminal Repeat) 5' et 3' et la région d'encapsidation et défectif pour l'ensemble des fonctions virales.

Par ailleurs, l'origine du vecteur adénoviral peut être variée aussi bien du point de vue de l'espèce que du sérotype. Il peut dériver du génome d'un adénovirus humain ou animal (canin, aviaire, bovin, murin, ovin, porcin, simien...) ou encore d'un hybride comprenant des fragments de génome adénoviral d'au moins deux origines différentes. On peut citer plus particulièrement les adénovirus canins CAV-1 et CAV-2, aviaire DAV ou encore bovine Bad (notamment de type 3) (Zakharchuk et al., Arch. Virol., 1993, *128*: 171-176 ; Spibey et Cavanagh, J. Gen. Virol., 1989, *70*: 165-172 ; Jouvenne et al., Gene. 1987, *60:* 21-28 ; Mittal et al., J. Gen. Virol., 1995, *76*: 93-102). Cependant, on prétérera un vecteur adénoviral d'origine humaine dérivant d'un adénovirus de sérotype C, notamment de type 2 ou 5.

L'invention a également pour objet un procédé de préparation d'une préparation virale contenant majoritairement des virus non enveloppés, ledit procédé comprenant au moins une étape d'inactivation de virus enveloppés selon le procédé de l'invention.

Avantageusement, le procédé de préparation selon l'invention comprend au moins :
(a) une étape de production de ladite préparation virale dans une lignée cellulaire appropriée,
(b) une étape de récolte de la préparation virale produite à l'étape (a) à partir de la lignée cellulaire productrice et/ou du surnageant de culture,
(c) de manière optionnelle, une étape de cassage des cellules de la lignée cellulaire productrice,
(d) de manière optionnelle, une étape de clarification,
(e) une étape d'inactivation de virus enveloppés telle que décrite ci-avant, et
(f) de manière optionnelle, une étape de purification.

Bien entendu, l'ordre des étapes peut varier, notamment pour ce qui est de l'étape d'inactivation (e), qui peut se situer immédiatement après la récolte des virus (étape b), après les étapes optionnelles c) ou d) ou encore être inclue dans l'étape de purification f).

Comme indiqué précédemment, l'étape (a) peut résulter de la transfection du génome du virus non enveloppé d'intérêt dans une lignée cellulaire appropriée. L'ADN viral introduit peut être le génome viral, éventuellement construit dans une bactérie (WO96/17070), dans une levure (WO95/03400) ou dans une cellule. La construction est réalisée par des techniques de biologie moléculaire ou de recombinaison homologue intermoléculaire classiques dans le domaine de l'art. L'ADN peut également être introduit dans la lignée cellulaire sous forme de fragments comprenant une partie du génome viral et présentant une région d'homologie permettant de reconstituer le génome complet par recombinaison entre les séquences homologues portées par chacun des fragments (Graham et Prevect, 1991, Methods in Molecular Biology, vol 7, p109-128 ; Ed Murey, The Human Press Inc.). Une autre alternative consiste à infecter la lignée cellulaire par un préstock viral. Les conditions d'infection peuvent être définies par l'homme de l'art. A titre illustratif, les cellules sont infectées avec le virus non enveloppé à une multiplicité d'infection (MOI) définie (environ 1 à 10 s'agissant d'un adénovirus défectif).

Après transfection ou infection, la culture est poursuivie de préférence à 37°C pendant un temps suffisamment long pour permettre l'amplification des virus. Selon la quantité de virus à produire, cette étape est réalisée en boîtes de culture, en fermenteur ou dans tout autre système de culture approprié. Généralement, la récolte des virus non enveloppé est effectuée entre 24 h et 1 semaine post infection ou transfection. Le temps de récolte peut être déterminé par plusieurs critères : titre viral optimal, observation d'une cytopathie (arrondissement des cellules productrices) et/ou réduction de la consommation d'oxygène. Une récolte à 48 h ou 72 h est préférée. Les virus sont recueillis soit à partir des cellules productrices soit à partir du surnageant de culture ou encore à partir de l'ensemble cellules et surnageant.

Dans le premier cas, les cellules productrices sont récoltées. Il est préférable de procéder à une étape de cassage des cellules, généralement après resuspension de la biomasse cellulaire, afin de libérer les virus produits de manière intracellulaire. Tous les moyens classiques peuvent être mis en oeuvre dans le cadre de l'invention, notamment les moyens chimiques et/ou mécaniques. On peut procéder par exemple à des cycles de congélation-décongélation qui fragilisent les membranes cellulaires, à une lyse enzymatique (emploi d'enzymes dégradant les membranes cellulaires) ou chimique (emploi de détergent, choc de pH, choc osmotique...). Les moyens mécaniques peuvent résulter d'ultrason (sonication), d'attrition (billes de verre DynoMill, BeadMill), de forces de pression et de cisaillement (homogéneiseur haute pression French Press), de microfluides (Microfluidics, Newton, MA) ou encore de l'action mécanique de deux cylindres générant des forces de cisaillement hydrauliques et mécaniques (homogéneiseur Silverson).

Lorsque la préparation virale est récoltée directement du milieu de culture, il n'est pas nécessaire de procéder à l'étape de cassage, le surnageant de culture pouvant être directement clarifié pour éliminer les débris cellulaires, par exemple par centrifugation à basse vitesse ou filtration en cascade. Dans ce cas, la culture peut être poursuivie pendant une durée plus longue afin de garantir un rendement maximal de virus.

Selon une troisième option, on peut récolter le surnageant et les cellules Dans ce cas, il convient de procéder à l'étape de cassage afin de libérer les virus intracellulaires et à l'étape de clarification.

L'étape de clarification a pour but d'éliminer les insolubles (débris cellulaire, floculats de macromolécules ...etc). Elle peut être réalisée par toute technique conventionnelle de filtration (filtration en profondeur, microfiltration tangentielle...) ou centrifugation (en continue...). Il peut être judicieux notamment lorsque la préparation virale est très concentrée d'éliminer la majorité des insolubles d'abord par centrifugation puis de poursuivre la clarification par filtration en profondeur. De nombreux filtres peuvent être utilisés dans le cadre de la présente invention à condition toutefois qu'ils aient une porosité permettant de laisser passer le virus non enveloppé d'intérêt et de retenir les insolubles. On indique que l'adénovirus a une taille d'environ 0,07 à 0,1 µm, nécessitant l'utilisation de filtres de porosité supérieure. Par ailleurs, les filtres peuvent être en matière synthétique (nylon), organique ( cellulose) ou non organique (zirconium). Selon un mode de réalisation avantageux, on procède à des filtrations successives sur des filtres de porosité décroissante, par exemple en premier lieu sur un filtre de porosité 8 µm (Sartorius 5591301P5--00) puis sur un filtre de porosité 5 µm (Sartorius 5591342P5--00) puis sur un filtre de porosité comprise entre 3 et 0,8 µm (Sartorius, Sartoclean CA capsule 5621304E9-00-A), puis sur un filtre de porosité comprise entre 0,8 et 0,65 µm (Sartorius, Sartoclean CA capsule 5621305G9-00-A). Selon une autre variante, la filtration peut être réalisée par microfiltration tangentielle sur membranes planes ou fibres creuses de porosité supérieure à la taille de l'adénovirus. A cet égard, les membranes Durapore (Millipore) et Omega (Pall) peuvent être employées.

L'étape de purification peut être réalisée par des techniques classiques antérieures, par exemple par ultracentrifugation (en gradient de chlorure de césium ou autre) ou chromatographie.

Selon un mode de réalisation avantageux, l'étape de purification du procédé de préparation selon l'invention comprend une étape de chromatographie, notamment par échange d'ions. De manière optionnelle, elle peut être combinée à une chromatographie d'un type différent, notamment par gel filtration afin de parfaire la purification des virus non enveloppés. Les deux chromatographies peuvent être réalisées dans un ordre quelconque, mais on préfère néanmoins procéder en premier lieu à la chromatographie échangeuse d'ions puis à la chromatographie de gel filtration.

Pour la chromatographie échangeuse d'ions, différents types de supports peuvent être utilisés tels que les supports à base de cellulose, d'agarose (gels Sepharose ou Macro-Prep), de dextran (gels Sephadex), d'acrylamide (gels Séphacryl, Trisacryl), de silice (gels TSK, SW), de poly(styrène-divinylbenzène) (gels Source ou Poros), de copolymères éthylène glycol-méthacrylate (gels Toyopearl HW, TSK, PW, fractogel EMD) ou des mélanges, notamment d'agarose et de dextran (gel Superdex). On retiendra plus particulièrement les supports agréés pour un usage humain ou vétérinaire par les autorités compétentes américaines (FDA pour food and drug administration) ou les agences de l'Union Européennne. En outre, le support retenu doit être lié, de préférence par liaison covalente, avec un ou plusieurs types de groupements susceptibles d'intéragir avec le virus non enveloppé à purifier (le support est dit fonctionnalisé). On préférera un groupement permettant un échange d'anions, notamment constitué d'amine ternaire ou quaternaire. Parmi les supports fonctionnalisés par des amines ternaires, on peut citer les résines fractogel-DEAE (diéthylaminoéthyl), fractogel DMAE (diméthylaminoéthyl) et le Toyopearl-DEAE. Parmi les supports fonctionnalisés par des amines quaternaires, on peut citer les résines Source Q, Mono Q, Q Sépharose, Poros HQ et QE, Streamline QXL (demande française 99 02167), les résines de type Fractogel TMAE et Toyopearl super Q. La résine Poros PI est un exemple approprié de support fonctionnalisé avec le polyéthylèneimine. Le support Fractogel-DEAE est préféré dans le cadre de la présente invention. La colonne est initialement équilibrée dans des conditions salines permettant la fixation des virus non enveloppés d'intérêt sur les groupements fonctionnels chargés positivement. Avantageusement, on utilise un tampon comprenant du NaCl à environ 250 mM final. Mais bien entendu, les conditions de chromatographie peuvent être adaptées en fonction de différents paramètres, notamment du volume de la colonne, du support choisi, du virus choisi et de la concentration virale. L'élution du virus retenu sur les groupes amines est réalisée en augmentant progressivement la concentration saline, de préférence à une concentration finale de 300 à 400 mM NaCl et, tout à fait préférentiellement entre 300 et 350 mM NaCl et les fractions comprenant le virus non enveloppé d'intérêt peuvent être déterminées par tous les moyens techniques de l'art (mesure spectrophotométrique de l'absorbance à 260 et 280 nm, visualisation de peptides ou génomes viraux ...). Il est également possible de connecter la colonne à un détecteur muni d'un filtre pour la détection en ligne des fractions virales. On indique que la fraction virale (composée d'ADN et de protéines) présente une absorbance caractéristique à 260 et 280 nm alors que les contaminants protéiques ne sont détectés qu'à 280 nm et les acides nucléiques libres à 260 nm.

Pour ce qui est de la chromatographie de gel filtration, le virus est purifié sur un support présentant un diamètre de billes compris entre 3 et 160 µm, avantageusement entre 5 et 105 µm et, de préférence entre 10 et 80 µm. De préférence, le support a une porosité proche de la taille du virus afin que celui-ci ne pénètre pas dans les billes. Au contraire, toutes les molécules de taille inférieure, vont pénétrer dans les billes et être retardées. Différents types de supports peuvent être utilisés tels que les matrices à base d'agarose (Sépharose), de dextran (gel Séphadex), d'acrylamide (gels Séphacryl et Trisacryl), la silice (gels TSK et SW), de copolymères éthylène glycol méthacrylate (gels Toyopearl HW, TSK et PW), et de mélanges , notamment d'agarose et de dextran (gel Superdex). Les supports mentionnés sont de préférence utilisés sans groupements de fonctionalisation. Les supports particulièrement appropriés à la mise en oeuvre du procédé de préparation selon l'invention sont les suivants :
- matrices allyl dextran-méthylène bis acrylamide (Séphacryl S300 HR de diamètre de bille compris entre 25 et 75 µm, Séphacryl S400 HR de diamètre de bille compris entre 25 et 75 µm, Séphacryl S500 HR de diamètre de bille compris entre 25 et 75 µm et Séphacryl S1000 SF de diamètre de bille compris entre 40 et 105 µm ; Pharmacia),
- matrices d'éthylène glycol-méthacrylate (Toyopearl HW 55, Toyopearl HW 65 et Toyopearl HW 75 de diamètre de billes variant de 20 à 60 µm ; Tosohaas),
- matrices de N acryl amine hydroxyl propanédiol (Trisacryl d'un diamètre de billes compris entre 80 et 160 µm ; Biosépra), et
- matrice d'agarose (Macro-Prep SE de diamètre de bille compris entre 20 et 80 µm ; Biorad).

A titre indicatif, le support de type Toyopearl HW65F ou HW65S (porosité 1000 Å) ou Séphacryl S400HR est préféré. La colonne est équilibrée dans un tampon présentant des conditions salines et un pH limitant les interactions hydrophobes entre le support et le virus. Avantageusement, on utilise un tampon Tris-HCl 50mM, MgCl₂ 2mM, saccharose 2% à pH 8,5. Les virus non enveloppés d'intérêt passent à travers les billes sans être retenus et sortent avant les contaminants de poids moléculaires inférieurs. Les fractions les contenant peuvent être déterminées par les techniques usuelles (absorbance à 260 et 280 nm, techniques d'électrophorèse, de PCR...). On notera qu'un avantage du procédé de préparation selon l'invention consiste en l'élimination au cours de cette étape (f) du solvant et de l'agent de solubilisation en usage dans le procédé d'inactivation selon l'invention. Selon un mode de réalisation optionnel, les fractions virales obtenues après l'étape de purification peuvent être rassemblées et éventuellement concentrées selon les techniques habituelles. On peut citer l'ultrafiltration tangentielle et la diafiltration. Les cassettes BioMax PES (Millipore référence PXB300C50 ou PXB01MC50) et PLCMK (Millipore référence PXC300C50) conviennent tout particulièrement.

En outre, le procédé de préparation selon l'invention peut comprendre des étapes supplémentaires et notamment une étape de dégradation des acides nucléiques (principalement d'origine cellulaire) présents en quantités importantes après le cassage des cellules. A cet effet, toutes les enzymes de restriction non spécifiques de type endo ou exonucléases peuvent être employées. Mais la méthode préférée consiste en un traitement par la benzonase. A titre indicatif, on utilise d'environ 5 à 50 U/ml de benzonase mais les conditions optimales peuvent être adaptées par l'homme du métier selon le volume à traiter et la viscosité de la préparation virale. L'action de la benzonase peut être appréciée par la réduction de la concentration en acides nucléiques en appliquant toute méthodologie divulguée dans la littérature. Bien que les étapes puissent être permutées, on préfère réaliser ladite étape de traitement à la benzonase entre les étapes de cassage (c) et de clarification (d) dudit procédé de préparation. Une autre alternative consiste à réaliser l'étape de traitement à la benzonase et l'étape d'inactivation de façon concomitante après les étapes de cassage et de clarification. En outre, la benzonase peut être utilisée en présence éventuellement de β cyclodextrine. Cette dernière aide à précipiter les lipides et peut être ajoutée à une concentration finale de 0,1 à 10 % et, en particulier, 1,5 %.

Le procédé de préparation selon l'invention peut également comprendre une étape de filtration stérilisante, ladite étape de filtration stérilisante étant de préférence réalisée après l'étape (f) dudit procédé de préparation. On aura avantageusement recours à des filtres de 0,22 µm d'une surface adaptée au volume à traiter. On peut citer, par exemple, les unités de filtration de type Minisart (Sartorius, reférence SM16534), Sartolab P20 (Sartorius, référence 18053D), Millex GF (Millipore, référence SLGS025BS), Millex GV (Millipore, référence SLGV025BS), Millex GP (Millipore, référence SLGPR25LS) ou encore Spirale Cap (Version Super CQS 92 HS ou HP ; Gelman Sciences), Criticap 50 (12995, Gelman Sciences) ou Millipak (Millipore ref. MPGL04SK2 ou MPGL02SH2). Puis, le filtrat peut être conditionné en doses ajustées à une concentration donnée.

La qualité, c'est à dire le degré de pureté de la préparation virale peut être suivie tout au long du procédé de préparation selon l'invention en déterminant la concentration résiduelle des contaminants et la fonctionnalité du virus non enveloppé d'intérêt. Dans le premier cas et s'agissant du mode de réalisation préféré, la disparition du Tween 80 (ou polysorbate 80) après l'étape (f) peut être appréciée par la méthode préconisée dans la pharmacopée européenne (1997, p 1372-1373) à l'aide de thiocyanate de potassium et chloroforme. La quantité de TNBP présente dans la préparation virale peut être titrée par la technique de chromatographie en phase gazeuse telle que divulguée dans Horowitz et al. (1985, Transfusion *25*, 516-522). La concentration résiduelle par les protéines peut être mesurée par toute technique de dosage des protéines. Une technique adéquate est celle du BCA (acide bicinchoninique) (kit Micro BCA Protein Assay Reagent Kit ; Pierce ref 23235). Pour ce qui est du principe actif viral, le nombre de particules totales est déterminé par spectrométrie à une longueur d'onde de 260 nm en présence de SDS (voir Shabram et al., 1997, Human Gene Therapy *8*, 453-465). La fonctionnalité du virus non enveloppé est généralement déterminée par sa capacité infectieuse, par exemple par titration du nombre d'unités infectieuses (voir Lusky et al., 1998, J. Virol. *72*, 2022-2032). Lorsqu'il s'agit d'un virus recombinant, on peut également évaluer l'expression du gène recombinant après infection d'une cellule cible, par fluorescence, méthodes immunologiques (ELISA, RIA...), méthodes immuno-enzymatiques (Western....), techniques de coloration ou luminescence....etc.

Le procédé de préparation selon l'invention s'adresse à des adénovirus. De préférence, ceux-ci présentent les caractéristiques définies ci-dessus.

Le choix des différentes lignées cellulaires appropriées à la mise en oeuvre du procédé selon l'invention est large et à la portée de l'homme de l'art. On choisira une lignée adaptée au virus non enveloppé retenu. S'agissant du mode de réalisation préféré (adénovirus recombinant défectif pour la réplication), on emploiera une lignée de complémentation adaptée aux déficiences de l'adénovirus telles que celles décrites dans la littérature. Il s'agit avantageusement d'une lignée complémentant la fonction E1, comme par exemple la lignée 293 provenant de cellules embryonnaires de rein humain et qui comprend intégrée dans son génome l'extrémité 5' du génome de l'Ad5 (Graham et al., 1977, J. Gen. Virol. *36*, 59-72). D'autres lignées complémentant E1 sont également disponibles (Imler et al., 1996, Gene Therapy *3*, 75-84 ; Fallaux et al., 1996, Human Gene Therapy *7*, 215-222 ; Fallaux et al., 1998, Human Gene Therapy *9*, 1909-1917). Lorsque les déficiences du virus concernent également les régions E2 ou E4, on peut avoir recours aux lignées de complémentation décrites dans Brough et al. (1992, Virology *190*, 624), Wang et al. (1995, Gene Therapy *2*, 775-783), Yeh et al. (1996, J. Virol. *70*, 559-565), Kougliak et Graham (1996, Human Gene Therapy *6*, 1575-1586) et Lusky et al. (1998, J. Virol. *72*, 2022-2032) et dans les demandes internationales WO94/28152 et WO97104119. Une autre alternative repose sur l'emploi d'un élément viral supplémentaire, désigné "virus auxiliaire" pour complémenter au moins en partie les fonctions défectives du virus non enveloppé d'intérêt. Les virus auxiliaires de l'art antérieur consistent en un génome viral, éventuellement délété d'une région essentielle pour laquelle le virus d'intérêt ne nécessite pas de complémentation ou qui est fournie par la lignée. De manière générale, une lignée de complémentation peut être générée par transfection des séquences virales restaurant la ou les fonctions défectives du virus, placées sous le contrôle des éléments nécessaires à leur expression dans une lignée cellulaire appropriée. A cet égard, elle peut être issue à partir d'une lignée cellulaire établie d'origine humaine ou animale et, de préférence, acceptable d'un point de vue pharmaceutique (utilisable pour la production à l'échelle industrielle de produits destinés à un usage humain et n'ayant pas de caractère pathogène connu). On peut citer entre autres les cellules KB, HeLa, Vero (ATCC CCL-81), BHK (ATCC CCL-10), A 549 (ATCC CCL-185), MRC5 (ATCC CCL-171), WI-38 (ATCC CCL-75), CHO, MDCK et MDBK. Une lignée appropriée dans le cadre de la présente invention peut également dériver d'une cellule primaire et en particulier de cellules de rétine ou de rein prélevées d'un embryon humain. On a de préférence recours à une lignée dérivant d'une cellule embryonnaire de rein humain, d'une cellule de rétine (notamment de rétine embryonnaire humaine HER) ou d'un carcinome humain (A549).

La présente invention concerne également une préparation virale obtenue selon le procédé de préparation selon l'invention ainsi qu'une cellule eucaryote infectée par une préparation virale selon l'invention. Il s'agit de préférence d'une cellule de mammifère et notamment humaine. Elle peut être primaire ou tumorale et d'une origine quelconque, notamment hématopoïétique (cellule souche totipotente, leucocyte, lymphocyte, monocyte ou macrophage...), musculaire (cellule satellite, myocyte, myoblaste, muscle lisse...), cardiaque, pulmonaire, trachéale, hépatique, épithéliale ou fibroblaste. On indique que la préparation de l'invention se distingue de celles de l'art antérieur en ce qu'elle est essentiellement dépourvue de virus enveloppés infectieux.

La présente invention concerne également une composition comprenant une préparation virale ou une cellule hôte selon l'invention. A titre de rappel, ladite préparation virale peut comprendre un ou plusieurs virus non enveloppés d'intérêt préparé(s) selon le procédé de l'invention. Ceux-ci peuvent être de la même famille (adénovirus portant un gène recombinant différent) ou non. Ladite composition est de préférence une composition pharmaceutique contenant au moins un véhicule acceptable d'un point de vue pharmaceutique.

Une composition selon l'invention peut être fabriquée de manière conventionnelle en vue d'une administration par voie locale, parentérale ou digestive. Les voies d'administration envisageables sont multiples. On peut citer par exemple la voie intra-gastrique, sous-cutanée, intracardiaque, intramusculaire, intraveineuse, intra-artérielle, intra-vasculaire, intra-péritonéale, intra-tumorale, intranasale, intrapulmonaire ou intratrachéale. Pour ces trois derniers modes de réalisation, une administration par aérosol ou instillation est avantageuse. L'administration peut avoir lieu en dose unique ou répétée une ou plusieurs fois après un certain délai d'intervalle. La voie d'administration et les doses de virus appropriées varient en fonction de divers paramètres, par exemple, de l'individu, de la pathologie, du gène d'intérêt à transférer, de la voie d'administration. A titre indicatif, les préparations à base de particules adénovirales peuvent être formulées sous forme de doses comprises entre 10⁴ et 10¹⁴ ufp (unités formant des plages), avantageusement 10⁵ et 10¹³ ufp et, de préférence, 10⁶ et 10¹² ufp.

La formulation peut également inclure un diluant, un adjuvant ou un excipient acceptable d'un point de vue pharmaceutique, de même que des agents de solubilisation, de stabilisation, de préservation. Une composition préférée est sous forme injectable. Elle peut être formulée en solution aqueuse, saline (phosphate, monosodique, disodique, magnésium, potassium....) ou isotonique. Le tampon de formulation décrit dans la demande internationale WO98/02522 convient tout particulièrement. Elle peut être présentée en dose unique ou en multidose sous forme liquide ou sèche (poudre, lyophilisat...) susceptible d'être reconstituée de manière extamporanée par un diluant approprié.

Une composition selon l'invention est plus particulièrement destinée au traitement préventif ou curatif de maladies par thérapie génique (y compris immunothérapie) et s'adresse plus particulièrement aux maladies prolifératives (cancers, tumeurs, dysplasies...etc), aux maladies infectieuses et notamment virales (induites par les virus de l'hépatite B ou C, le HIV, l'herpès, les rétrovirus....etc), aux maladies génétiques (mucoviscidose, dystrophine, hémophilie, diabète...) et aux maladies cardiovasculaires (resténose, ischémie, dislipidémie...).

La présente invention est également relative à l'utilisation thérapeutique ou prophylactique d'une préparation virale d'une cellule hôte, d'une composition ou d'une composition pharmaceutique selon l'invention, pour la préparation d'un médicament destiné au transfert et à l'expression d'un gène d'intérêt dans une cellule ou un organisme hôte. Le médicament est plus particulièrement destiné au traitement des maladies par thérapie génique. Selon une première possibilité, il peut être administré directement *in vivo* (par exemple par injection intraveineuse, dans une tumeur accessible, dans les poumons par aérosol, dans le système vasculaire au moyen d'une sonde appropriée...). On peut également adopter l'approche *ex vivo* qui consiste à prélever des cellules du patient (cellules souches de la moëlle osseuse, lymphocytes du sang périphérique, cellules musculaires...), de les transfecter ou infecter *in vitro* selon les techniques de l'art et de les réadminister au patient après une étape d'amplification éventuelle. La prévention et le traitement de nombreuses pathologies peuvent être envisagés. Une utilisation préférée consiste à traiter ou prévenir les cancers, tumeurs et maladies résultant d'une prolifération cellulaire non désirée. Parmi les applications envisageables, on peut citer les cancers du sein, de l'utérus (notamment ceux induits par les papillomas virus), de la prostate, du poumon, de la vessie, du foie, du colon, du pancréas, de l'estomac, de l'oesophage, du larynx du système nerveux central et du sang (lymphomes, leucémie etc...). Elle est également utile dans le cadre des maladies cardiovasculaires, par exemple pour inhiber ou retarder la prolifération des cellules de muscles lisses de la paroi vasculaire (resténose). Enfin pour ce qui est des maladies infectieuses, l'application au SIDA peut être envisagée.

L'invention s'étend également à une méthode pour le traitement des maladies par thérapie génique, caractérisée en ce que l'on administre à un organisme ou à une cellule hôte ayant besoin d'un tel traitement une préparation virale, une cellule hôte ou une composition selon l'invention.

### EXEMPLES

La présente invention est illustrée, sans pour autant être limitée, par les exemples suivants.

Les adénovirus recombinants ont été construits par la technique de recombinaison homologue décrite dans Chartier et al. (1996, J. Virol. 70, 4805-4810). Les constructions mises en oeuvre ont été réalisées selon les techniques générales de génie génétique et de clonage moléculaire, détaillées dans Maniatis et al., (1989, Laboratory Manual, Cold Spring Harbor, Laboratory Press, Cold Spring Harbor, NY ou une édition plus récente) ou selon les recommandations du fabricant lorsqu'on utilise un kit commercial. Les étapes de clonage utilisent la souche *E. coli* 5K (hsdR, mcrA), DH5α[(recA1, endA1, hodR17 (r-m-), supE44 thi-1, gyrA (nalr)] ou NM522 (supE, thi, Δ(lac-proAB), hsd5, (r-m-), (F = proAB, lacI^{q}, ZΔM15) et celles de recombinaison homologue la souche *E. coli* BJ 5183 (Hanahan, 1983, J. Mol. Biol. *166*, 557-580). S'agissant de la réparation des sites de restriction, la technique employée consiste en un remplissage des extrémités 5' protubérantes à l'aide du grand fragment de l'ADN polymérase I d'*E*. *coli* (Klenow, Boehringer Mannheim). Les fragments d'ADN sont purifiés à l'aide du kit de purification d'ADN GeneCleanII^{R} (Bio101Inc.). Par ailleurs, les fragments de génome adénoviral employés dans les différentes constructions sont indiqués précisément selon leur position dans la séquence nucléotidique du génome de l'Ad5 telle que divulguée dans la banque de données Genebank sous la référence M73260.

En ce qui concerne la biologie cellulaire, les cellules sont transfectées ou transduites et cultivées selon les techniques standards bien connues de l'homme du métier. On a recours aux lignées cellulaires 293 (ATCC CRL-1573), A549 E1+ (WO94/28152) et 293-E4ORF6+7 (Lusky et al., 1998, J. Virol. *72*, 2022-2032). Il est entendu que d'autres lignées cellulaires peuvent être utilisées. Les cellules sont maintenues en culture à 37°C en atmosphère humide enrichie à 5% de CO₂ dans du milieu DMEM (Dulbecco's Modified Eagle Medium, Gibco BRL) complémenté avec 1 mM de glutamine, 1% d'acides aminés (Gibco BRL), 40 µg/l de gentamycine et 10% de sérum de veau foetal (SVF,Gibco BRL). Les cellules peuvent également être produites en réacteur de culture cellulaire. Les cellules sont transfectées selon les techniques de l'art (précipitation au phosphate de calcium...). Le titre en unités infectieuses (iu) ou particules virales totales est déterminé selon les techniques de l'art (Lusky et al., 1998, J. Virol. *72*, 2022-2032)

Les exemples qui suivent ont été réalisés à l'aide d'adénovirus recombinants exprimant un gène marqueur ou un gène thérapeutique. Ils sont dérivés du sérotype Ad5 et ont la structure suivante :
- AdTG6297 est un vecteur adénoviral de première génération défectif pour les fonctions E1 (délétion des nt 459 à 3328) et E3 (délétion du fragment XbaI s'étendant des nt 28592 à 30470) dans le génome duquel est inséré en remplacement de la région E1, une cassette d'expression du gène marqueur codant pour la protéine GFP (pour green fluorescent protein). Celle-ci réagit à l'excitation lumineuse (485 nm) par l'émission d'une lumière fluorescente dont on mesure l'intensité au moyen d'un filtre (535 nm). Plus précisément, la cassette est composée du promoteur CMV suivi d'un intron chimère, de la séquence codant pour la protéine GFP et le polyA du virus SV40. Les séquences introniques sont isolées du plasmide pCI (Promega Corp, pCI mammalian expression vecteur E1731) et comprennent le site donneur d'épissage de l'intron 1 du géne β-globine humaine ainsi que le point de branchement et le site accepteur d'épissage du gène d'une immunoglobine de souris. Les particules virales sont produites par transfection du vecteur AdTG6297 dans une lignée de complémentation de E1 (293 ou A549 E1+) et amplifiées par passages successifs sur une lignée permissive (complémentant E1).
- Le vecteur AdTG5643 est un vecteur de seconde génération délété des régions E1 (nt 459 à 3328), E3 (nt 28592 à 30470) et E4 (nt 32994 à 34998) et exprimant le gène thérapeutique CFTR humain. La cassette d'expression est constituée du promoteur précoce CMV, de l'ADNc CFTR et du poly A du gène β-globine de lapin et est insérée à la place des séquences E1 délétées. Les particules virales sont produites par transfection du vecteur AdTG5643 dans une lignée de complémentation de E1 et E4 (293-E4ORF6+7) et un stock viral constitué par passages successifs sur une lignée permissive (complémentant E1 et E4).
- Le vecteur AdTG13383 est un vecteur délété des régions E1 (nt459 à 3511) et E3 (nt 28539 à 30470) et exprimant le gène thérapeutique IL2 humain. La cassette d'expression insérée à la place des séquences E1, est constituée du promoteur précoce CMV, de l'intron synthetique isolé du plasmide pCI, de l'ADNc codant pour l'IL2 humaine et du poly A SV40. Les particules virales sont produites par transfection du vecteur pTG13383 dans une lignée de complémentation de E1 et un stock viral constitué par passages successifs sur une lignée permissive (complémentant E1).

### EXEMPLE 1 : Préparation de virus à partir des cellules de complémentation.

Les cellules A549-E1+ sont cultivées en boîtes de culture jusqu'à atteindre une densité cellulaire de 2,5.10⁵ cellules/cm² et sont ensuite infectées avec un préstock d'AdTG6297 à raison d'une MOI d'environ 3. Les cellules infectées sont récoltées à 72 h post infection et centrifugées à basse vitesse. Le culot est repris dans environ 600 ml de milieu de culture sans sérum. La préparation virale ainsi obtenue correspond à un volume d'environ 20 l.

Le virus intracellulaire est libéré après cassage des cellules soumises à l'action mécanique pendant 7 à 10 min d'un homogéneiseur Silvérson (L4R-Silverson) réglé à une vitesse de rotation de 4200 tours/min.

A ce stade, la préparation virale est très visqueuse du fait de la libération de l'ADN génomique suite au cassage cellulaire. On ajoute à la préparation virale un volume d'un tampon permettant une action optimale de la benzonase et constitué de Tris 100 mM, MgCl₂ 4 mM, saccharose 4% pH 8,5 auxquels a été ajouté l'agent de solubilisation Tween 80 (Merck référence 8-22187-1000) à une concentration de 2%. Le mélange est mis sous agitation à température ambiante avant d'ajouter la benzonase à raison de 50 U/ml (Merck référence 101697) et on laisse la réaction se poursuivre pendant 1 à 2 h à température ambiante et sous agitation.

La préparation virale ainsi traitée est ensuite clarifiée par filtration en profondeur en quatre étapes successives. La première filtration est effectuée à travers des filtres de 8 µm (Sartorius 5591301P5--00) puis sur des filtres de 5 µm (Sartorius 5591342P5--00) puis sur des filtres de 3 à 0,8 µm (Sartoclean CA capsule 5621304E9-00-A) et suivie d'une quatrième filtration à travers des filtres de 0,8 à 0,65 µm (Sartoclean CA capsule 5621305G9-00-A).

L'étape d'inactivation des virus enveloppés est réalisée par action du TNBP à une concentration finale de 0,3 %. Pour ce faire, le filtrat est dilué volume à volume dans une solution tampon de Tris 50 mM, MgCl₂ 2 mM, saccharose 2%, NaCl 350 mM et TNBP 0,6% (Aldrich 24-049-4), pH 8,5. Il est également possible d'ajouter à la préparation virale filtrée 9 volumes d'un tampon plus concentré (Tris 50 mM, MgCl₂ 2 mM, saccharose 2%, NaCl 1,82 M et TNBP 3 %, pH 8,5). Il est à remarquer que les conditions salines utilisées (NaCl 250 mM final) correspondent aux conditions d'équilibration de la chromatographie par échange d'ions. On laisse l'action du TNBP/Tween 80 se poursuivre sous agitation (500 rpm) à température ambiante pendant 3h ou à 4°C pendant 4 h.

Pour l'étape de chromatographie par échange d'ions, la préparation virale inactivée est chargée sur une colonne contenant du fractogel EMD DEAE (Merck, référence 1,16883), préalablement équilibrée avec du tampon Tris 50 mM, MgCl₂ 2 mM, saccharose 2%, NaCl 250 mM, pH 8,5. Après rinçage avec le tampon d'équilibrage, les constituants adsorbés sur le support sont élués avec le tampon précédent en présence de concentrations croissantes de sel (NaCl 300 mM, 350 mM, 400 mM ...etc.). Un débit de 30 à 100 cm/h et, de préférence 50 cm/h est appliqué. Les différentes fractions éluées sont visualisées par mesure de l'absorbance à 260 et 280 nm. Généralement, les protéines (détectées à 280 nm uniquement) sont éluées par le tampon contenant 300 mM en NaCl. Le second pic d'élution (détecté à 260 et 280 nm) contient les adénovirus d'intérêt qui sont élués à une concentration saline de 350 mM. La colonne est régénérée en présence de NaCl 1,5M. Le fractogel est régulièrement sanitisé par passage de NaOH 0,5N.

La fraction virale est ensuite chargée sur une colonne contenant du gel Toyopearl HW-65F (Tosohaas, référence 43304 ou 07465) ou 65S (Tosohaas, référence 43354 ou 07467) ou du Séphacryl S400HR (Pharmacia, référence 17-0609-10) préalablement équilibrée avec du tampon Tris 25 mM, MgCl₂ 2 mM, saccharose 2%, pH 8,5. Généralement, le volume de préparation virale injecté correspond à 5 à 20 % du volume de la colonne de gel filtration et le débit appliqué varie de 5 à 100 cm/h avec une préférence pour 10 à 50 cm/h. Le profil d'élution suivi par la mesure de l'absorbance à 280 nm, montre que le pic d'adénovirus est le premier pic obtenu en sortie de colonne.

L'étape suivante consiste à diafiltrer la préparation virale afin de pouvoir la conditionner dans le tampon de formulation. A cette fin, les fractions virales sont rassemblées et on mesure le titre en particules virales totales et unités infectieuses sur une aliquote. Si le titre viral est suffisant, les virus sont dilués dans le tampon de formulation, soumis à une filtration stérilisante sur filtre de 0,22 m (Sartolab P20, Sartorius référence 18053D) et répartis en doses. Si le titre est trop faible, la préparation virale peut être préalablement concentrée par ultrafiltration tangentielle et/ou diafiltration à l'aide par exemple des cassettes BioMax PES (Millipore référence PXB300C50) et PLCMK (Millipore référence PXC300C50).

Dans une expérience représentative effectuée à partir d'une préparation virale d'AdTG6297 exprimant le marqueur GFP, le bilan en titre viral est le suivant :

| Etapes | ui totales x 10¹¹ | Rendement (%) |
|---|---|---|
| Départ | 35 | 100 |
| Benzonase | 81,6 | 233 |
| Filtration | 48,2 | 138 |
| Inactivation t0 | 110 | 314 |
| Inactivation t 4h | 154 | 440 |
| Chromato Fractogel-DEAE Flow through | 14,8 | |
| Elution Nacl 350mM | 30 | 85 |
| - ui représente le nombre d'unités infectieuses. | | |
| - le flow through représente le matériel qui n'est pas retenu sur la colonne et qui est donc directement élué | | |

L'augmentation des titres en adénovirus d'un facteur 3 à 4 lors de l'étape d'inactivation peut s'expliquer par une désagrégation des virus en présence du solvant.

### EXEMPLE 2 : Préparation de virus à partir de la culture cellulaire.

L'exemple 1 est reproduit à la différence que l'on récolte 72 h post infection les cellules et le surnageant de culture (volume d'environ 20 l) et l'ensemble est directement soumis à l'étape de cassage.

### EXEMPLE 3 : Inactivation de virus enveloppés

### 3.1 Validation sur une préparation de rétrovirus

L'efficacité du procédé d'inactivation proposé dans la présente invention est évaluée sur des rétrovirus recombinants exprimant le gène marqueur LacZ codant pour l'enzyme β-galactosidase. Une culture de 20 F500 de cellules 293 est préparée. Après centrifugation 8 min à 3000 rpm, les cellules sont reprises dans du milieu sans sérum. La préparation contient 3x10⁷ cellules /ml dans un volume de 25 ml. Les cellules sont cassées au Silverson puis centrifugées 10 min à 3500 rpm pour éliminer les débris. La préparation est alors séparée en 2, une première moitié étant diluée volume à volume dans le tampon benzonase (Tris 100 mM, MgCl₂ 4 mM, saccharose 4%, pH 8,5) en absence de β-cyclodextrine alors que la seconde moitié est traitée de manière similaire mais en présence de 3% de β-cyclodextrine (1,5 % final). Les échantillons sont clarifiés par filtration en cascade sur filtres Minisart (Sartorius) de 5 µm (référence 17594Q), de 1,2 µm (référence 17593Q) et 0,8 µm (référence 17592Q). Chaque échantillon est alors traité avec un volume de Tris 50 mM, MgCl₂ 2 mM, saccharose 2%, NaCl 450 mM, TNBP 0,6% et Tween 80 2%, pH 8,5. On introduit les particules rétrovirales à une concentration finale de 1,5 x 10⁶ particules infectieuses/ml. Le titre en particules rétrovirales est déterminé après 15 sec, 20 min, 1h, 2h et 4 h d'incubation soit à 4°C soit à température ambiante. Le titrage est effectué par comptage des cellules bleues selon la méthodologie standard (voir par exemple US 5,747,323).

Les résultats sont résumés ci-après :
avant traitement : 1,5x10⁶ particules de rétrovirus/ml
température ambiante, + β-cyclodextrine, 15 sec d'incubation : <1x10³/ml
température ambiante, - β-cyclodextrine, 15 sec d'incubation :1x10⁴/ml 4°C, + β-cyclodextrine, 15 sec d'incubation : <1x10³/ml

Au delà de 15 sec d'incubation, les titres de particules rétrovirales sont inférieurs au seuil de détection (10³ particules infectieuses/ml). Les résultats montrent que le procédé d'inactivation de l'invention permet une réduction de l'infectivité des rétrovirus de 2 logs en 15 sec. La présence de β-cyclodextrine est avantageuse car elle améliore d'un facteur supplémentaire de 10 l'inactivation rétrovirale.

### 3.2 Validation d'une préparation d'adénovirus contaminée par le virus BVD.

On prépare une préparation adénovirale à petite échelle (18 ml) selon le protocole utilisé à l'exemple 1. Après clarification par filtration en profondeur en 4 étapes successives, on introduit 2 ml d'une solution de particules de BVD. Puis on procède à l'étape d'inactivation en présence d'une concentration finale de TNBP 0,3 % et Tween 80 1 %. Le titre en particules BVD et adénovirales infectieuses est déterminé après 0 min, 15 min, 60 min et 120 min d'incubation à température ambiante.

Nous obtenons ainsi une cinétique d'inactivation du virus BVD :

| Temps | Titre (log₁₀ TCID50) |
|---|---|
| T0 | 5.88 |
| T 5 sec | 5.27 |
| T 15 min | 3.87 |
| T 60 min | < 2.57 |
| T 120 min | 1.18 |

Soit une réduction de 4.7 Log₁₀, après 2 heures d'inactivation.

### EXEMPLE 4 : Préparation de virus à partir des cellules de complémentation.

Les cellules de complémentation pour la fonction adénovirale E1 sont cultivées en bioréacteur en milieu Excell 525 (JRH Biosciences) jusqu'à atteindre une concentration de 1x10⁶ cellules/ml et sont ensuite infectées avec un volume équivalent d'un préstock d'AdTG13383 à raison d'une MOI d'environ 10. Les cellules infectées sont récoltées à 72 h post infection. Le surnageant de culture (volume d'environ 20 l) et l'ensemble est directement soumis à l'étape de cassage pour obtenir la préparation virale brute à purifier.

Les particules virales intracellulaires sont libérées après cassage des cellules soumises à l'action mécanique pendant 7 à 10 min d'un homogéneiseur Silverson (275 UHLS) réglé à une vitesse de rotation de 50 Hz (vitesse de 8.1).

L'étape de clarification est réalisée par filtrations successives sur filtres de porosité décroissante, en premier lieu sur un filtre de porosité 8 µm (Sartopure 300PP2 5592501) puis sur un filtre de porosité 5 µm (Sartopure 300 PP3 5592542) enfin sur un filtre de porosité comprise entre 3 et 0,8 µm (Sartorius, Sartoclean CA capsule 5621304E9-00-A).

On ajoute à la préparation virale clarifiée un volume d'un tampon permettant une action optimale de la benzonase et constitué de Tris-HCl 100 mM, MgCl₂ 4 mM, saccharose 4% pH 8,5 comprenant en outre du Tween 80 (Merck référence 8-22187-1000) à une concentration de 2 %. Le mélange est mis sous agitation à température ambiante avant d'ajouter la benzonase à raison de 10 U/ml (Merck référence 101697) et on laisse la réaction se poursuivre pendant 2 h à température ambiante et sous agitation (500 tr/min). On peut également soumettre la préparation virale clarifiée à l'action concomitante de la benzonase (étape de dégradation de l'ADN) et du TNBP / Tween 80 (inactivation des virus enveloppés). Pour ce faire, le TNBP (Aldrich 24-049-40) est ajouté à une concentration finale de 0.3% à la préparation précédente. L'action du TNBP / Tween 80 se poursuit sous agitation (500 tr/min) pendant 2 heures à température ambiante. Le titre en unités infectieuses déterminé après chaque étape majeure du procédé est résumé dans le tableau suivant :

| Etapes | Rendement UI - % (global) | Rendement UI - % (étape) |
|---|---|---|
| Cassage des cellules | 100 | - |
| Clarification | 96 | 96 |
| Dnase / Inactivation | 130 | 135 |

L'augmentation des titres en adénovirus d'un facteur 1.3 lors de l'étape d'inactivation peut s'expliquer par une désagrégation des virus en présence du solvant.

### EXEMPLE 5 : Validation d'une préparation d'adénovirus contaminée par le virus VSV.

On prépare une préparation adénovirale à petite échelle selon le protocole utilisé à l'exemple 4. Après cassage et clarification par filtration en profondeur, on introduit dans la préparation adénovirale (2,5 x 10¹⁰ ui) une solution de particules de VSV (ATCC VR-158 ; 9,9 log₁₀ TCTID50). Puis on procède à l'étape d'inactivation en présence d'une concentration finale de TNBP 0,3 % et Tween 80 1 % en même temps que l'étape de dégradation des acides nucléiques en présence de 10 U/ml de Benzonase (Merck référence 101697). Le titre en particules VSV infectieuses est déterminé sur cellules VERO selon les techniques de l'art (Virology Methods Manual 1996, pp. 35-40, Ed. Mahy and Kangro, Academic Press Ltd. London) après 0 min, 30 min, 60 min et 120 min d'incubation à température ambiante.

| Temps | Titre (log₁₀ TCID50/ml) |
|---|---|
| T0 | 9.2 |
| T 15 min | 6.8 |
| T 60 min | 5.3 |
| T 120 min | 1.7 |

Soit une réduction de 7.5 Log₁₀ après 2 heures d'inactivation.

Dans leur ensemble, les données montrent que le procédé de l'invention permet d'inactiver les virus enveloppés d'une préparation d'adénovirus recombinant sans nuire à leur infectivité et avec un rendement global supérieur à 100%.

## Revendications

1. Procédé d'inactivation de virus enveloppés contaminant une préparation virale contenant majoritairement des adénovirus, dans lequel :
- on introduit dans ladite préparation virale une quantité suffisante de solvant tri-n butyl phosphate (TNBP) comprise entre 0,05 % et 1 % (volume/volume),
- on laisse agir ledit TNBP à une température comprise entre + 4°C et + 37°C, à un pH compris entre 6,5 et 8,5, pendant un temps suffisamment long pour réduire de manière significative la quantité de virus enveloppés contaminant ladite préparation virale,
ledit procédé d'inactivation conservant le pouvoir infectieux desdits adénovirus.

2. Procédé d'inactivation de virus enveloppés selon la revendication 1, dans lequel la quantité de TNBP introduite dans ladite préparation virale est comprise entre 0,1 % et 0,6 % (volume/volume).

3. Procédé d'inactivation de virus enveloppés selon la revendication 2, dans lequel le TNBP est introduit dans ladite préparation virale en une quantité de 0,3 % (volume/volume).

4. Procédé d'inactivation de virus enveloppés selon l'une des revendications 1 à 3, mis en oeuvre en présence d'un agent de solubilisation, ledit agent de solubilisation étant un détergent, à une concentration comprise entre 0,01 % et 5 % (volume/volume).

5. Procédé d'inactivation de virus enveloppés selon la revendication 4, dans lequel ledit détergent est un détergent non ionique.

6. Procédé d'inactivation de virus enveloppés selon la revendication 5, dans lequel ledit détergent non ionique est un Tween.

7. Procédé d'inactivation de virus enveloppés selon la revendication 6, dans lequel ledit Tween est le Tween 80.

8. Procédé d'inactivation de virus enveloppés selon l'une des revendications 4 à 7, dans lequel la quantité de détergent introduite dans ladite préparation virale est comprise entre 0,1 et 2 % (volume/volume).

9. Procédé d'inactivation de virus enveloppés selon l'une des revendications 1 à 8, dans lequel on laisse agir ledit TNBP en présence éventuellement dudit détergent, à une température comprise entre + 15°C et + 25°C.

10. Procédé d'inactivation de virus enveloppés selon l'une des revendications 1 à 9, dans lequel on laisse agir ledit TNBP en présence éventuellement dudit détergent, à un pH de 8,5.

11. Procédé d'inactivation de virus enveloppés selon l'une des revendications 1 à 10, dans lequel on laisse agir ledit TNBP en présence éventuellement dudit détergent pendant un temps compris entre 1 h et 5 h.

12. Procédé d'inactivation de virus enveloppés selon l'une des revendications 1 à 11, mis en oeuvre sous agitation.

13. Procédé d'inactivation de virus enveloppés selon l'une des revendications 7 à 12, dans lequel :
- on introduit dans ladite préparation virale du TNBP à une concentration finale comprise entre 0,1 % et 0,6 % (volume/volume) et du Tween 80 à une concentration finale comprise entre 0,5 % et 2 % (volume/volume),
- on laisse agir ledit TNBP et ledit Tween 80 à la température ambiante,
- à un pH de 8,5, et
- pendant un temps compris entre 1 heure et 5 heures.

14. Procédé d'inactivation de virus enveloppés selon l'une des revendications 1 à 13, réalisé dans des conditions de conductivité comprises entre 5 et 500 mS/cm.

15. Procédé d'inactivation de virus enveloppés selon la revendication 14, dans lequel les conditions de conductivité sont comprises entre 10 et 100 mS/cm.

16. Procédé d'inactivation selon l'une des revendications 1 à 15, dans lequel ledit adénovirus est recombinant.

17. Procédé de préparation selon l'une des revendications 1 à 16, dans lequel ledit adénovirus est défectif pour la réplication.

18. Procédé de préparation d'une préparation virale contenant majoritairement des adénovirus comprenant au moins une étape d'inactivation de virus enveloppés conformément au procédé défini dans l'une quelconque des revendications 1 à 17.

19. Procédé de préparation selon la revendication 18, comprenant au moins :
(a) une étape de production de ladite préparation virale dans une lignée cellulaire appropriée,
(b) une étape de récolte de la préparation virale produite à l'étape (a) à partir de la lignée cellulaire productrice et/ou du surnageant de culture, et
(c) de manière optionnelle, une étape de cassage des cellules de la lignée cellulaire productrice.

20. Procédé de préparation selon la revendication 19, comprenant au moins :
(a)une étape de production de ladite préparation virale dans une lignée cellulaire appropriée,
(b)une étape de récolte de la préparation virale introduite à l'étape (a) à partir de la lignée cellulaire productrice et/ou du surnageant de culture,
(c)de manière optionnelle, une étape de cassage des cellules de la lignée cellulaire productrice,
(d)de manière optionnelle, une étape de clarification,
(e)une étape d'inactivation de virus enveloppés selon l'une des revendications 1 à 17, et
(f) de manière optionnelle, une étape de purification.

21. Procédé de préparation selon la revendication 20, dans lequel l'étape de purification comprend une étape de chromatographie.

22. Procédé de préparation selon la revendication 21, dans lequel l'étape de chromatographie comprend une chromatographie échangeuse d'ions puis une chromatographie de gel filtration.

## Claims

1. Method of inactivating enveloped viruses contaminating a viral preparation predominantly containing adenoviruses, in which:
- a sufficient quantity of solvent tri-n-butyl phosphate (TNBP) of between 0.05% and 1% (volume/volume) is introduced into the said viral preparation,
- the said TNBP is allowed to act at a temperature of between about +4°C and +37°C, at a pH of between 6.5 and 8.5 for a period which is sufficiently long to significantly reduce the quantity of enveloped viruses contaminating the said viral preparation,
the said method of inactivation preserving the infectivity of the said adenoviruses.

2. Method of inactivating enveloped viruses according to Claim 1, in which the quantity of TNBP introduced into the said viral preparation is between 0.1% and 0.6% (volume/volume).

3. Method of inactivating enveloped viruses according to Claim 2, in which the TNBP is introduced into the said viral preparation in a quantity of 0.3% (volume/volume).

4. Method of inactivating enveloped viruses according to one of Claims 1 to 3, carried out in the presence of a solubilizing agent, the said solubilizing agent being a detergent, at a concentration of between 0.01% and 5% (volume/volume).

5. Method of inactivating enveloped viruses according to Claim 4, in which the said detergent is a nonionic detergent.

6. Method of inactivating enveloped viruses according to Claim 5, in which the said nonionic detergent is a Tween.

7. Method of inactivating enveloped viruses according to Claim 6, in which the said Tween is Tween 80.

8. Method of inactivating enveloped viruses according to one of Claims 4 to 7, in which the quantity of detergent introduced into the said viral preparation is between 0.1 and 2% (volume/volume).

9. Method of inactivating enveloped viruses according to one of Claims 1 to 8, in which the said TNBP is allowed to act, optionally in the presence of the said detergent, at a temperature of between +15°C and +25°C.

10. Method of inactivating enveloped viruses according to one of Claims 1 to 9, in which the said TNBP is allowed to act, optionally in the presence of the said detergent, at a pH of 8.5.

11. Method of inactivating enveloped viruses according to one of Claims 1 to 10, in which the said TNBP is allowed to act, optionally in the presence of the said detergent, for a period of between 1 h and 5 h.

12. Method of inactivating enveloped viruses according to one of Claims 1 to 11, carried out with stirring.

13. Method of inactivating enveloped viruses according to one of Claims 7 to 12, in which:
- there are introduced into the said viral preparation TNBP at a final concentration of between 0.1% and 0.6% (volume/volume) and Tween 80 at a final concentration of between 0.5% and 2% (volume/volume),
- the said TNBP and the said Tween 80 are allowed to act at room temperature,
- at a pH of 8.5, and
- for a period of between 1 hour and 5 hours.

14. Method of inactivating enveloped viruses according to one of Claims 1 to 13, carried out under conductivity conditions of between 5 and 500 mS/cm.

15. Method of inactivating enveloped viruses according to Claim 14, in which the conductivity conditions are between 10 and 100 mS/cm.

16. Method of inactivation according to one of Claims 1 to 15 in which the said adenovirus is recombinant.

17. Method of preparation according to one of Claims 1 to 16, in which the said adenovirus is replication defective.

18. Method of preparing a viral preparation predominantly containing adenoviruses comprising at least one step of inactivating enveloped viruses according to the method defined in any one of Claims 1 to 17.

19. Method of preparation according to Claim 18, comprising at least:
(a) one step for producing the said viral preparation in an appropriate cell line,
(b) one step for harvesting the viral preparation produced in step (a) from the producing cell line and/or from the culture supernatant, and
(c) optionally, one step for breaking the cells of the producing cell line.

20. Method of preparation according to Claim 19, comprising at least:
(a) one step for producing the said viral preparation in an appropriate cell line,
(b) one step for harvesting the viral preparation introduced in step (a) from the producing cell line and/or from the culture supernatant,
(c) optionally, one step for breaking the cells of the producing cell line,
(d) optionally, one clarification step,
(e) one step for inactivating enveloped viruses according to one of Claims 1 to 17, and
(f) optionally, one purification step.

21. Method of preparation according to Claim 20, in which the purification step comprises a chromatography step.

22. Method of preparation according to Claim 21, in which the chromatography step comprises an ionexchange chromatography and then a gel filtration chromatography.

## Patentansprüche

1. Verfahren zur Inaktivierung von umhüllten Viren, die ein Viruspräparat kontaminieren, das hauptsächlich Adenoviren enthält, bei dem man:
- in das Viruspräparat eine ausreichende Menge des Lösungsmittels Trin-butylphosphat (TNBP) zwischen 0,05 % und 1,0 % (Volumen/Volumen) einschließlich einführt,
- das TNBP bei einer Temperatur zwischen + 4 °C und + 37 °C einschließlich bei einem pH zwischen 6,5 und 8,5 einschließlich über eine ausreichend lange Zeit einwirken lässt, um signifikant die Menge an umhüllten Viren, die das Viruspräparat kontaminieren, zu verringern,
wobei das Verfahren zur Inaktivierung das Infektionsvermögen der Adenoviren bewahrt.

2. Verfahren zur Inaktivierung von umhüllten Viren nach Anspruch 1, in dem die Menge an TNBP, die in das Viruspräparat eingeführt wird, zwischen 0,1 % und 0,6 % (Volumen/Volumen) einschließlich liegt.

3. Verfahren zur Inaktivierung von umhüllten Viren nach Anspruch 2, in dem das TNBP in das Viruspräparat in einer Menge von 0,3 % (Volumen/Volumen) eingeführt wird.

4. Verfahren zur Inaktivierung von umhüllten Viren nach einem der Ansprüche 1 bis 3, das in Anwesenheit eines Solubilisierungsmittels durchgeführt wird, wobei das Solubilisierungsmittel ein Detergens bei einer Konzentration zwischen 0,01 % und 5 % (Volumen/Volumen) einschließlich ist.

5. Verfahren zur Inaktivierung von umhüllten Viren nach Anspruch 4, in dem das Detergens ein nichtionisches Detergens ist.

6. Verfahren zur Inaktivierung von umhüllten Viren nach Anspruch 5, in dem das nichtionische Detergens ein Tween ist.

7. Verfahren zur Inaktivierung von umhüllten Viren nach Anspruch 6, in dem das Tween Tween 80 ist.

8. Verfahren zur Inaktivierung von umhüllten Viren nach einem der Ansprüche 4 bis 7, in dem die Detergensmenge, die in das Viruspräparat eingeführt wird, zwischen 0,1 und 2 % (Volumen/Volumen) einschließlich liegt.

9. Verfahren zur Inaktivierung von umhüllten Viren nach einem der Ansprüche 1 bis 8, in dem man das TNBP gegebenenfalls in Anwesenheit des Detergens bei einer Temperatur zwischen + 15 °C und + 25 °C einschließlich einwirken lässt.

10. Verfahren zur Inaktivierung von umhüllten Viren nach einem der Ansprüche 1 bis 9, in dem man das TNBP gegebenenfalls in Anwesenheit des Detergens bei einem pH von 8,5 einwirken lässt.

11. Verfahren zur Inaktivierung von umhüllten Viren nach einem der Ansprüche 1 bis 10, in dem man das TNBP gegebenenfalls in Anwesenheit des Detergens über eine Zeit zwischen 1 h und 5 h einschließlich einwirken lässt.

12. Verfahren zur Inaktivierung von umhüllten Viren nach einem der Ansprüche 1 bis 11, das unter Rühren durchgeführt wird.

13. Verfahren zur Inaktivierung von umhüllten Viren nach einem der Ansprüche 7 bis 12, in dem man:
- in das Viruspräparat TNBP mit einer Endkonzentration zwischen 0,1 % und 0,6 % (Volumen/Volumen) einschließlich und Tween 80 mit einer Endkonzentration zwischen 0,5 % und 2 % (Volumen/Volumen) einschließlich einführt,
- das TNBP und das Tween 80 bei Umgebungstemperatur
- bei einem pH von 8,5 und
- über eine Zeit zwischen 1 Stunde und 5 Stunden einschließlich einwirken lässt.

14. Verfahren zur Inaktivierung von umhüllten Viren nach einem der Ansprüche 1 bis 13, das unter Leitfähigkeitsbedingungen zwischen 5 und 500 mS/cm einschließlich durchgeführt wird.

15. Verfahren zur Inaktivierung von umhüllten Viren nach Anspruch 14, in dem die Leitfähigkeitsbedingungen zwischen 10 und 100 mS/cm einschließlich liegen.

16. Verfahren zur Inaktivierung nach einem der Ansprüche 1 bis 15, in dem das Adenovirus rekombinant ist.

17. Verfahren zur Inaktivierung nach einem der Ansprüche 1 bis 16, in dem das Adenovirus replikationsdefekt ist.

18. Verfahren zur Herstellung eines Viruspräparats, das hauptsächlich Adenoviren enthält, welches mindestens einen Schritt der Inaktivierung von umhüllten Viren gemäß einem Verfahren umfasst, das in irgendeinem der Ansprüche 1 bis 17 definiert ist.

19. Verfahren zur Herstellung nach Anspruch 18, mindestens umfassend:
(a) einen Schritt der Produktion des Viruspräparats in einer geeigneten Zelllinie,
(b) einen Schritt der Ernte des Viruspräparats, das im Schritt (a) produziert wurde, ausgehend von der Produzenten-Zelllinie und/oder dem Kulturüberstand und
(c) gegebenenfalls einen Schritt des Aufbrechens der Zellen der erzeugenden Zelllinie.

20. Verfahren zur Herstellung nach Anspruch 19, mindestens umfassend:
(a) einen Schritt der Produktion des Viruspräparats in einer geeigneten Zelllinie,
(b) einen Schritt der Ernte des Viruspräparats, das im Schritt (a) produziert wurde, ausgehend von der Produzenten-Zelllinie und/oder dem Kulturüberstand,
(c) gegebenenfalls einen Schritt des Aufbrechens der Zellen der erzeugenden Zelllinie,
(d) gegebenenfalls einen Klärungsschritt,
(e) einen Schritt der Inaktivierung von umhüllten Viren nach einem der Ansprüche 1 bis 17 und
(f) gegebenenfalls einen Reinigungsschritt.

21. Verfahren zur Herstellung nach Anspruch 20, in dem der Reinigungsschritt einen Chromatographieschritt umfasst.

22. Verfahren zur Herstellung nach Anspruch 21, in dem der Chromatographieschritt eine lonenaustausch-Chromatographie, dann eine Gelfiltrations-Chromatographie umfasst.
